# EUROPEAN PATENT APPLICATION

(11) **EP 1 611 886 A2**
(43) Date of publication of application: **04.01.2006**
(21) Application number: 05017162.8
(22) Date of filing: 30.08.2000
(51) Int. Cl.: A61K 31/355, A61K 31/403, A61K 31/616, A61K 45/06, A61P 9/04, A61P 9/10, A61P 9/12

(54) **Inhibitors of the renin-angiotensin system for the prevention of cardiovascular disorders**

(30) Priority: 30.08.1999 US 151436 P
(62) Divisional of application: 00965906.1
(71) Applicant: Sanofi-Aventis Deutschland GmbH, 65929 Frankfurt am Main (DE)
(72) Inventor: Schoelkens, Bernward, 65779 Kelkheim (DE); Bender, Norbert, 67098 Bad Dürckheim (DE); Rangoonwala, Badrudin, 65719 Hofheim (DE); Yusuf, Salim, Carlisle, Ontario L0R 1H2 (CA); Dagenais, Gilles, St.-Nicholas, Quebec, J7A 3P8 (CA); Gerstein, Hertzel, Hamilton, Ontario, L8S 4A8 (CA)

(57) **Abstract**

The present invention relates to the use of an inhibitor of the renin-angiotensin system or a pharmaceutically acceptable derivative thereof, optionally together with an other antihypertensive, a cholesterol lowering agent, a diuretic or aspirin, in the manufacture of a medicament for the prevention of cardiovascular events; to a method of preventing cardiovascular events comprising administering to a patient in need of such prevention an effective amount of an inhibitor of the renin angiotensin system or a pharmaceutically acceptable derivative thereof, optionally together with an other antihypertensive, a cholesterol lowering agent, a diuretic or aspirin; or to a combination product containing an an inhibitor of the renin-angiotensin system or a pharmaceutically acceptable derivative thereof and a cholesterol lowering agent.

## Description

### FIELD OF THE INVENTION

The present invention relates to the use of an inhibitor of the renin-angiotensin system or a pharmaceutically acceptable derivative thereof, optionally together with an other antihypertensive, a cholesterol lowering agent, a diuretic or aspirin, in the manufacture of a medicament for the prevention of cardiovascular events; to a method of preventing cardiovascular events comprising administering to a patient in need of such prevention an effective amount of an inhibitor of the renin angiotensin system or a pharmaceutically acceptable derivative thereof, optionally together with an other antihypertensive, a cholesterol lowering agent, a diuretic or aspirin; or to a combination product containing an an inhibitor of the renin-angiotensin system or a pharmaceutically acceptable derivative thereof and a cholesterol lowering agent.

### BACKGROUND OF THE INVENTION

The renin-angiotensin system (RAS) can be inferred with by inhibition of the enzymes synthesizing angiotensins or by blocking the corresponding receptors at the effector sites. There are many marketed or investigation-stage agents which inhibit RAS activity, and many fall into two broad classes: the inhibitors of angiotensin-converting enzyme (ACE), whose approved names generally end in "pril" or in the case of active metabolites "-prilat", and antagonists at angiotensin receptors (more specifically, currently, the AT₁ receptor) (Angiotensin II Antagonists), whose approved names generally end in "-sartan". Also potentially of increasing importance may be a class of drugs known as neutral endopeptidase (NEP) inhibitors which will also have an ACE-inhibitory effect or the potential to reduce RAS activity and are therefore also known as NEP/ACE-inhibitors.

ACE inhibitors are well known in the art for their activity in inhibiting angiotensin converting enzyme, thereby blocking conversion of the decapeptide angiotensin I to angiotensin II. The principal pharmacological and clinical effects of ACE inhibitors arise from suppression of synthesis of angiotensin II. Angiotensin II is a potent pressor substance and, therefore, blood pressure lowering can result from inhibition of its biosynthesis, especially in animals and humans whose hypertension is angiotensin II related. ACE inhibitors are effective antihypertensive agents in a variety of animal models and are clinically useful for the treatment of hypertension in humans.

ACE inhibitors are also employed for the treatment of heart conditions such as hypertension and heart failure. It is known that at least some ACE inhibitors can improve (i.e., decrease) morbidity and mortality in patient populations with heart conditions, ie. patients with low ejection fraction (EF) or heart failure (HF), but their role in a broader population of high risk patients without ventricular dysfunction or HF is unknown.

### SUMMARY OF THE INVENTION

The present invention generally relates to the use of an inhibitor of the renin-angiotensin system or a pharmaceutically acceptable derivative thereof in the manufacture of a medicament for the prevention of cardiovascular events.

The present inventions further relates to the use of an inhibitor of the renin-angiotensin system or a pharmaceutically acceptable derivative thereof in the manufacture of a medicament for the prevention of myocardial infarction (MI), worsening of angina and cardiac arrest.

Furthermore, the present inventions relates the use of an inhibitor of the renin-angiotensin system or a pharmaceutically acceptable derivative thereof in the manufacture of a medicament for the prevention of cardiovascular events such as for example myocardial infarction (MI), worsening of angina or cardiac arrest in a patient with an increased cardiovascular risk, for example due to a manifest coronory heart disease, a history of transient ischaemic attacks or stroke, or a history of peripheral vascular disease.

More generally, the present inventions relates the use of an inhibitor of the renin-angiotensin system or a pharmaceutically acceptable derivative thereof in the manufacture of a medicament for the prevention of cardiovascular events in patients with no evidence of left ventricular dysfunction or heart failure.

The present invention further relates the use of an inhibitor of the renin-angiotensin system or a pharmaceutically acceptable derivative thereof in the manufacture of a medicament for the prevention of myocardial infarction (MI), stroke, cardiovascular death or overt nephropathie in a diabetic patient.

Another embodiment of the present invention is the use of an inhibitor of the renin-angiotensin system or a pharmaceutically acceptable derivative thereof together with an other antihypertensive, a cholesterol lowering agent, a diuretic or aspirin in the manufacture of a medicament for the prevention of cardiovascular events, for example stroke, congestive heart failure, cardiovascular death, myocardial infarction, worsening of angina, cardiac arrest, or revascularisation procedures.

Yet another embodiment of the present invention is the use of an inhibitor of the renin-angiotensin system or a pharmaceutically acceptable derivative thereof together with an other antihypertensive, a cholesterol lowering agent, a diuretic or aspirin in the manufacture of a medicament for the prevention of diabetes or diabetic complications.

A further embodiment of the present invention is the use of an inhibitor of the renin-angiotensin system or a pharmaceutically acceptable derivative thereof together with an other antihypertensive, a cholesterol lowering agent, a diuretic or aspirin in the manufacture of a medicament for the prevention of congestive heart failure (CHF) in a patient not previously having congestive heart failure.

Another embodiment of the present invention is a combination product containing an inhibitor of the renin-angiotensin system or a pharmaceutically acceptable derivative thereof and an other antihypertensive, a cholesterol lowering agent, a diuretic or aspirin for the use in the prevention of cardiovascular events.

Yet another embodiment of the present invention is a combination product containing an inhibitor of the renin-angiotensin system or a pharmaceutically acceptable derivative thereof and a cholesterol lowering agent.

A further embodiment of the present invention is a method of preventing cardiovascular events, for example myocardial infarction, worsening of angina and cardiac arrest, comprising administering to a patient in need of such prevention an effective amount of an inhibitor of the renin-angiotensin system or a pharmaceutically acceptable derivative thereof, and particular in patients having an increased cardioavascular risk.

An other embodiment of the present invention is a method of preventing myocardial infarction, stroke, cardiovascular death or overt nephropathie in a diabetic patient, comprising administering to said patient an effective amount of an inhibitor of the renin-angiotensin system or a pharmaceutically acceptable derivative thereof.

A further embodiment of the present invention is a method of preventing cardiovascular events, for example stroke, congestive heart failure, cardiovascular death, myocardial infarction, worsening of angina, cardiac arrest, or revascularisation procedures, or diabetes or diabetic complications comprising administering to a patient in need of such prevention an effective amount of an inhibitor of the renin-angiotensin system or a pharmaceutically acceptable derivative thereof together with an effective amount of an other antihypertensive, a cholesterol lowering agent, a diuretic or aspirin (combination therapy).

Yet another embodiment of the present invention is a method of preventing congestive heart failure in a patient not previously having congestive heart failure, comprising administering to said patient an effective amount of an inhibitor of the renin-angiotensin system or a pharmaceutically acceptable derivative thereof together with an effective amount of an other antihypertensive, a cholesterol lowering agent, a diuretic or aspirin (combination therapy).

### DETAILED DESCRIPTION OF THE INVENTION

It has been surprisingly found that cardiovascular events such as stroke, congestive heart failure, cardiovascular death, myocardial infarction, worsening of angina, cardiac arrest, or revascularisation procedures such as coronary atery bypass graft surgery (CABG), PTCA, Peripheral Angioplasty Surgery, Amputation, Cariotid Endarterectomy) and metabolic disorders such as diabetis or diabetic complications such as overt nephropathy, renal dialysis or laser therapy, or new microalbuminuria can be prevented in a broad population of high risk patients with no evidence of left venticular dysfunction or heart failure, by use of an inhibitor of the RAS system.

Furthermore and very surprisingly, the prevention of such cardiovascular events is also observed in a very broad range of high risk patients in addition to other effective therapies with for example antihypertensives (other than inhibitors of the RAS system), diuretics, cholesterol lowering agents or aspirin.

Thus the present invention describes a new method to prevent cardiovascular events, comprising administering to a patient in need of such prevention an effective amount of an inhibitor of the renin angiotensin system or a pharmaceutically acceptable derivative thereof, optionally together with an other antihypertensive, a cholesterol lowering agent, a diuretic or aspirin.

High risk patients are for instance those patients which are at risk having an cardiovascular event due to a manifest coronary heart disease, a history of transient ischaemic attacks or stroke, or a history of peripheral vascular disease.
Another group of high risk patients include those patients with diabetis.

The phrase "diabetis" as used herein includes both type I diabetis, also known as insulin -dependent, diabetis mellitus (IDMM), and type II diabetis, also known as non-insulin-dependent diabetis mellitus (NIDDM).

The phrase "diabetic complications" as used herein includes overt nephropathy, need for laser therapy or dialysis).

The phrase "inhibitor of the renin-angiotensin system (RAS) or a pharmaceutically accetable derivative thereof" as used herein includes any compound which by itself or upon administration blocks the negative effects of angiotensin II on the vasculature either by reducing the synthesis of angiotensin II or blocking its effect at the receptor.

Inhibitors of the RAS include ACE inhibitors, Angiotensin II antagonist and renin inhibitors and the pharmaceutically accetable derivatives thereof including prodrugs and metabolites.

The phrase "angiotensin converting enzyme inhibitor" ("ACE inhibitor") is intended to embrace an agent or compound, or a combination of two or more agents or compounds, having the ability to block, partially or completely, the rapid enzymatic conversion of the physiologically inactive decapeptide form of angiotensin ("Angiotensin I") to the vasoconstrictive octapeptide form of angiotensin ("Angiotensin II"). The phrase "ACE inhibitor" also embraces so-called NEP/ACE inhibitors (also referred to as selective or dual acting neutral endopeptidase inhibitors) which possess neutral endopeptidase (NEP) inhibitory activity and angiotensin converting enzyme (ACE) inhibitory activity.

Example of ACE inhibitors suitable for use herein are for instance the following compounds: AB-103, ancovenin, benazeprilat, BRL-36378, BW-A575C, CGS-13928C, CL242817, CV-5975, Equaten, EU-4865, EU-4867, EU-5476, foroxymithine, FPL 66564, FR-900456, Hoe-065, I5B2, indolapril, ketomethylureas, KRI-1177, KRI-1230, L681176, libenzapril, MCD, MDL-27088, MDL-27467A, moveltipril, MS-41, nicotianamine, pentopril, phenacein, pivopril, rentiapril, RG-5975, RG-6134, RG-6207, RGH0399, ROO-911, RS-10085-197,'RS-2039, RS 5139, RS 86127, RU-44403, S-8308, SA-291, spiraprilat, SQ26900, SQ-28084, SQ-28370, SQ-28940, SQ-31440, Synecor, utibapril, WF-10129, Wy-44221, Wy-44655, Y-23785, Yissum, P-0154, zabicipril, Asahi Brewery AB-47, alatriopril, BMS 182657, Asahi Chemical C-111, Asahi Chemical C-112, Dainippon DU-1777, mixanpril, Prentyl, zofenoprilat, 1
(- (I-carboxy-6- (4-piperidinyl) hexyl) amino) -1-oxopropyl octahydro-IH-indole-2-carboxylic acid, Bioproject BP1.137, Chiesi CHF 1514, Fisons FPL-66564, idrapril, perindoprilat and Servier S-5590, alacepril, benazepril, captopril, cilazapril, delapril, enalapril, enalaprilat, fosinopril, fosinoprilat, imidapril, lisinopril, perindopril, quinapril, ramipril, ramiprilat, saralasin acetate, temocapril, trandolapril, trandolaprilat, ceranapril, moexipril, quinaprilat and spirapril.

A group of ACE inhibitors of high interest are alacepril, benazepril, captopril, cilazapril, delapril, enalapril, enalaprilat, fosinopril, fosinoprilat, imidapril, lisinopril, perindopril, quinapril, ramipril, ramiprilat, saralasin acetate, temocapril, trandolapril, trandolaprilat, ceranapril, moexipril, quinaprilat and spirapril.

Many of these ACE inhibitors are commercially available, especially those listed in the above group. For example, a highly preferred ACE inhibitor ramipril (known from EP 79022) is sold by Aventis, e.g. under the trademark Delix® or Altace®. Enalapril or Enalapril Maleate, and Lisinopril are two more highly preferred ACE inhibitors sold by Merck & Co. Enalapril is sold under the trademark Vasotec®. Lisinopril is sold under the trademark Prinivil®.

Examples of NEP/ACE inhibitors suitable for use herein inclose those disclosed in U.S. Patents Nos. 5,508,272, 5,362,727, 5,366,973, 5,225,401, 4,722,810, 5223,516, 5,552,397, 4,749,688, 5,504,080, 5,612,359, 5,525,723, 5,430,145, and 5,679,671, and European Patent Applications 0481522, 0534263, 0534396, 0534492 and 0671172.

Preferred are those NEP/ACE inhibitors which are designated as preferred in the above U.S. patents and European Patent Applications and are incorporarted herein by reference. Especially preferrred is the NEP/ACE inhibitor omapatrilat (disclosed in U.S. Patent No. 5,508,272, or MDL100240 (disclosed in U.S. Patent No. 5,430,145).

The phrase "angiotensin II antagonist" is intended to embrace an agent or compound, or a combination of two or more agents or compounds, having the ability to block, partially or completely the binding of angiotensin II at angiotensin receptors, specifically at the AT₁ receptor.

### Example of Angiotensin II Antagonists suitable for use herein are for instance the following compounds:

Saralasin acetate, candesartan cilexetil, CGP-63170, EMD-66397, KT3-671, LR-B/081, valsartan, A-81282, BIBR-363, BIBS-222, BMS-184698, candesartan, CV-11194, EXP-3174, KW-3433, L-161177, L-162154, LR-B/057, LY-235656, PD-150304, U-96849, U-97018, UP-275-22, WAY-126227, WK-1492.2K, YM-31472, losartan potassium, E-4177, EMD-73495, eprosartan, HN-65021, irbesartan, L-159282, ME-3221, SL-91.0102, Tasosartan, Telmisartan, UP-269-6, YM-358, CGP-49870, GA-0056, L-159689, L-162234, L-162441, L-163007, PD-123177, A-81988, BMS-180560, CGP-38560A, CGP-48369, DA-2079, DE-3489, DuP-167, EXP-063, EXP-6155, EXP-6803, EXP-7711, EXP-9270, FK-739, HR-720, ICI-D6888, ICI-D7155, ICI-D8731, isoteoline, KRI-1177, L-158809, L-158978, L-159874, LR B087, LY-285434, LY-302289, LY-315995, RG-13647, RWJ-38970, RWJ-46458, S-8307, S-8308, saprisartan, saralasin, Sarmesin, WK-1360, X-6803, ZD-6888, ZD-7155, ZD-8731, BIBS39, CI-996, DMP-811, DuP-532, EXP-929, L-163017, LY-301875, XH-148, XR-510, zolasartan and PD-123319.

A group of Angiotensin II Antagonists of high interest are saralasin acetate, candesartan cilexetil, valsartan, candesartan, losartan potassium, eprosartan, irbesartan, tasosartan, or telmisartan.

Examples of renin inhibitors suitable for use herein are for instance the following compounds: enalkrein; RO 42-5892; A 65317; CP 80794; ES 1005; ES 8891; SQ 34017; CGP 29287; CGP 38560; SR 43845; U-71038; A 62198; A 64662, A-69729, FK 906 and FK 744.

Pharmaceutically acceptable derivatives of RAS inhibitors are understood to include physiologically tolerable salts of RAS inhibitors, such physiologically tolerable salts are understood as meaning both their organic and inorganic salts, such as are described in Remington's Pharmaceutical Sciences (17th Edition, page 1418 (1985)). On account of the physical and chemical stability and the solubility, for acidic groups, inter alia, sodium, potassium, calcium and ammonium salts are preferred; for basic groups, inter alia, salts of hydrochloric acid, sulfuric acid, phosphoric acid or of carboxylic acids or sulfonic acids, such as, for example, acetic acid, citric acid, benzoic acid, maleic acid, fumaric acid, tartaric acid and p-toluenesulfonic acid are preferred.

The RAS inhibitors suitable for use herein or their pharmaceutically acceptable derivatives can be used in animals, preferably in mammals, and in particular in human, as pharmaceuticals per se, in mixtures with one another or in the form of pharmaceutical preparations.

The present invention also relates to pharmaceutical formulations comprising as active ingredient at least one RAS inhibitor and/or an pharmaceutically acceptable derivative thereof in addition to customary pharmaceutically innocuous excipients and auxiliaries and their use in the prevention of cardiac events and the production of medicaments therefor. The pharmaceutical preparations normally contain 0.1 to 99 percent by weight, preferably 0.5 to 95 percent by weight, of the RAS inhibitor and/or an pharmaceutically acceptable derivative thereof. The pharmaceutical preparations can be prepared in a manner known per se. To this end, the RAS inhibitor and/or an pharmaceutically acceptable derivative thereof are brought, together with one or more solid or liquid pharmaceutical excipients and/or auxiliaries and, if desired, in combination with other pharmaceutical active compounds into a suitable administration form or dose form, which can then be used as a pharmaceutical in human medicine or veterinary medicine.

Pharmaceuticals which contain a RAS inhibitor and/or an pharmaceutically acceptable derivative thereof can be administered orally, parenterally, intravenously, rectally or by inhalation, the preferred administration being dependent on the particular symptoms of the disorder. The RAS inhibitors and/or an pharmaceutically acceptable derivative thereof can be used here on their own or together with pharmaceutical auxiliaries, namely both in veterinary and in human medicine.

The person skilled in the art is familiar on the basis of his expert knowledge with the auxiliaries which are suitable for the desired pharmaceutical formulation. In addition to solvents, gel-forming agents, suppository bases, tablet auxiliaries and other active compound excipients, it is possible to use, for example, antioxidants, dispersants, emulsifiers, antifoams, flavor corrigents, preservatives, solubilizers or colorants.

For an oral administration form, the active compounds are mixed with the additives suitable therefor, such as excipients, stabilizers or inert diluents and are brought by means of the customary methods into the suitable administration forms, such as tablets, coated tablets, hard capsules, aqueous, alcoholic or oily solutions. Inert excipients which can be used are, for example, gum arabic, magnesia, magnesium carbonate, potassium phosphate, lactose, glucose or starch, in particular corn starch. Preparation can take place here both as dry and as moist granules. Possible oily excipients or solvents are, for example, vegetable or animal oils, such as sunflower oil or codliver oil.

For subcutaneous or intravenous administration, the active compounds are brought into solution, suspension or emulsion, if desired with the substances customary therefor such as solubilizers, emulsifiers or other auxiliaries. Suitable solvents, for example, are: water, physiological saline solution or alcohols, e.g. ethanol, propanol, glycerol, and additionally also sugar solutions such as glucose or mannitol solutions, or alternatively a mixture of the various solvents mentioned.

Pharmaceutical formulations suitable for administration in the form of aerosols or sprays are, for example, solutions, suspensions or emulsions of the active compound of the formula I in a pharmaceutically acceptable solvent, such as, in particular, ethanol or water, or a mixture of such solvents.

If required, the formulation can also contain other pharmaceutical auxiliaries such as surfactants, emulsifiers and stabilizers, and also a propellant. Such a preparation customarily contains the active compound in a concentration from approximately 0.1 to 10, in particular from approximately 0.3 to 3, % by weight.

The dose of the active compound to be administered and the frequency of administration will depend on the potency and duration of action of the compounds used; additionally also on the nature of the indication and on the sex, age, weight and individual responsiveness of the mammal to be treated.

On average, the daily dose in a patient weighing approximately 75 kg is at least 0.001 mg/kg, preferably 0.01 mg/kg, to about 20 mg/kg, preferably 1 mg/kg, of body weight.

The RAS inhibitors and/or an pharmaceutically acceptable derivative thereof can also be used to achieve an advantageous theraupeutic action together with other pharmacologically active compounds for the prevention of the abovementioned syndromes.

The present invention furthermore relates to a combination product containing an inhibitor of the renin-angiotensin system or a pharmaceutically acceptable derivative thereof and an other antihypertensive, a cholesterol lowering agent, a diuretic or aspirin for the use in the prevention of cardiovascular events.

The invention additionally relates very generally to the combination of a RAS inhibitor and/or an pharmaceutically acceptable derivative thereof with a cholosterol lowering agent.

In addition to administration as a fixed combination, the invention also relates to the simultaneous, separate or sequential administration of an RAS inhibitor and/or an pharmaceutically acceptable derivative thereof with an other antihypertensive, a cholesterol lowering agent, a diuretic or aspirin.

The invention additionally relates to a pharmaceutical preparation comprising an RAS inhibitor and/or an pharmaceutically acceptable derivative thereof and a cholesterol lowering agent (combination product).

The pharmaceutical preparations of the combination product according to the invention can be prepared, for example, by either intensively mixing the individual components as a powder, or by dissolving the individual components in the suitable solvent such as, for example, a lower alcohol and then removing the solvent.

The weight ratio of the RAS inhibitor and/or an pharmaceutically acceptable derivative thereof and the cholesterol lowering agent in the novel combinations and preparations lies in the range from 1:0.01 to 1:100, preferably 1:0.1 to 1:10.

The novel combinations and preparations in total may contain 0.5-99.5% by weight, in particular 4-99% by weight, of these active compounds.

When used according to the invention in mammals, preferably in human, the doses of the various active compound components, for example, vary in the range from 0.001 to 100 mg/kg/day.

Typically, the individual daily dosages for these combinations can range from about one-fifth of the minimally recommended clinical dosages to the maximum recommended levels for the entities when they are given singly.

By means of combined administration, the effect of one combination component can be potentiated by the other respective component, i.e. the action and/or duration of action of a novel combination or preparation is stronger or longer lasting than the action and/or duration of action of the respective individual components (synergistic effect). This leads on combined administration to a reduction of the dose of the respective combination component, compared with individual administration. The novel combinations and preparations accordingly have the advantage that the amounts of active compound to be administered can be significantly reduced and undesired side effects can be eliminated or greatly reduced.

A preferred combination product would contain for instance as a RAS inhibitors alacepril, benazepril, captopril, cilazapril, delapril, enalapril, enalaprilat, fosinopril, fosinoprilat, imidapril, lisinopril, perindopril, quinapril, ramipril, ramiprilat, saralasin acetate, temocapril, trandolapril, trandolaprilat, ceranapril, moexipril, quinaprilat or spirapril, most preferably ramipril and as a cholosterol lowering agent lovastatin, pravastatin, simvastatin or fluvastatin.

The phrase "combination therapy", in defining use of an inhibitor of the RAS system together with an other antihypertensive, a cholosterol lowering agent, a diuretic or aspirin is intended to embrace administration of each agent in a sequential manner in a regimen that will provide beneficial effects of the drug combination, and is intended as well to embrace co-administration of these agents in a substantially simultaneous manner, such as by oral ingestion of a single capsule having a fixed ratio of these active agents or ingestion of multiple, separate capsules for each agent.

"Combination therapy" will also include simultaneous or sequential administration by intravenous, intramuscular or other parenteral routes into the body, including direct absorption through mucuous membrane tissues, as found in the sinus passages. Sequential administration also includes drug combination where the individual elements may be administered at different times and/or by different routes but which act in combination to provide a beneficial effect.

The phrase "effective amount "is intended to qualify the amount of each agent for use in the combination therapy which will achieve the goal of preventing cardiac events while avoiding adverse side effects typically associated with each agent.

Examples of classes of other antihypertensives for use in the combination product or useful in the combination therapy are for example calcium channel blockers (or calcium antagonists) and beta-blockers.

Useful beta-blockers include timolol, atenolol, metoprolol, propanolol, nadolol and pindololpropanolol.

Useful calicum channel blockers include diltiazem, felodipine, nifedipine, amlodipine, nimodipine, isradipine, nitrendipine and verapamil.

Useful diuretics include methyclothiazide, hydrochlorothiazide, torsemide, metolazone, furosemide, chlorthalidone, N-(5-sulfamoyl-1,3,4-thiadiazol-2-yl)acetamide, triamterene, chlorothiazide, indapamide, bumetanide, amiloride, spironolactone, bendroflumethiazide, benzthiazide, cyclothiazide, quinethazone, hydroflumethiazide, polythiazide, trichlormethiazide, and ethacrynic acid.

An example for useful cholesterol lowering agents are statins.

The conversion of 3-hydroxy-Omethylglutaryl-coenzyme A (HMGCoA) to mevalonate is an early and rate-limiting step in the cholesterol biosynthetic pathway. This step is catalyzed by the enzyme HMOCoA reductase. Statins inhibit HMGCoA reductase from catalyzing this conversion. As such, statins are collectively potent cholesterol lowering agents.

Statins include such compounds as simvastatin, disclosed in U.S. 4,444,784, pravastatin, disclosed in U.S. 4,346,227, cerivastatin, disclosed in U.S. 5,502,199 mevastatin, disclosed in U.S. 3,983,140, velostatin, disclosed in U.S. 4,448,784 and U.S. 4,450,171; fluvastatin, disclosed in U.S. 4,739,073, compactin, disclosed in U.S. 4,804,770; lovastatin, disclosed in U.S. 4,231,938; dalvastatin, disclosed in EP-A 738510, fluindostatin, disclosed in EP-A 363934; atorvastatin, disclosed in U.S. 4,681,893, atorvastatin calcium, disclosed in U.S. 5,273,995; and dihydrocompactin, disclosed in U.S. 4,450,171, all of the above mentioned documents being incorporated herein by reference.

Preferred statins include lovastatin, pravastatin, simvastatin and fluvastatin.

Aspirin irreversibly inactivates platelet cyclooxygenase by acetylating this enzyme at the active site. In addition to reducing mortality, aspirin also reduces strokes and myocardial infarction. The excact mechanisms of the benefit of aspirin is not known.

### EXAMPLES

The examples as set forth herein are meant to exemplify the various aspects of carrying out the present invention and are not intended to limit the invention in any way.

A large-scale clinical trial (HOPE (Heart Outcomes Prevention Evaluation) Study) was designed to examine the effect of the ACE inhibitor ramipril versus placebo in reducing cardiovascular events. 9,297 high risk patients (≥ 55 yrs with evidence of vascular disease or diabetes plus one additional risk factor), without known low EF or HF were randomized to receive Ramipril (2.5mg to 10mg/day) or matching placebo for a mean duration of 5 years. The primary outcome was the first occurrence of the composite of cardiovascular (CV) mortality, myocardial infarction or stroke. The study was stopped at 4.5 years by the independent Data and Safety Monitoring Board because of convincing evidence of benefit.
646 (13.9%) patients allocated to Ramipril and 816 (17.5%) placebo patients experienced a primary outcome (Relative risk, RR of 0.78, 95% confidence interval of 0.70-0.86; Portable=0.000002). There were clear and significant reductions separately in CV deaths (6.0% v. 8.0%, RR of 0.75, Portable=0.0002), myocardial infarction (9.8% v. 12.0%, RR of 0.68, Portable=0.0002). Secondary outcomes such as total mortality (10.3% v. 12.2%, RR of 0.00035), revascularization procedures (16.0% v. 18.4%, RR of 0.85, Portable=0.0013), cardiac arrests (0.8% v. 1.2%, RR of 0.63; Portable=0.03), heart failure (7.4% v. 9.4%, RR of 0.78; Portable=0.0005), and diabetic complications (6.4% v. 7.7%, RR 0.85; Portable=0.017), were also significantly reduced. Other outcomes included worsening or new angina, or new heart failure (irrespective of hospitalization).

Ramipril significantly reduces mortality, myocardial infarction, stroke, revascularization procedures, and heart failure and prevents diabetic complications in a broad range of high risk patients without low EF or heart failure.

The protocols and results are set forth in the Examples presented hereinbelow.

### Example 1

### Study Design

In a double blind, 2x2 factorial, randomized trial, HOPE evaluated Ramipril or vitamin E in 9541 patients. A substudy of 244 patients tested a low dose (2.5 mg/day) versus the full dose (10 mg/day) of Ramipril. The primary outcome in these 244 patients is reported as a footnote to Table 3. Therefore the main results report is on 9,297 patients randomized to receive 10mg Ramipril or equivalent placebo. The effects of vitamin E are reported separately. The design of HOPE has been published (Can J Cardiology 1996; 12(2); 127-137), a brief summary follows below:

### Patient inclusion/exclusion

Men an women were eligible if aged 55 years and older, with prior coronary artery disease, stroke, peripheral vascular disease or diabetes plus at least one other risk factor (current or previous hypertension, elevated total cholesterol, low HDL cholesterol, current cigarette smoking, known microalbuminuria or previous vascular disease). Patents who had HF, were known to have low EF, those on ACE-I or vitamin E, those with uncontrolled hypertension or overt nephropathy, or recent MI (<4 weeks) were excluded. In this large, simple trial it was impractical to measure left ventricular function in all patients (none of whom had heart failure or were considered to need an ACE-I). Instead, echocardiograms were done in all patients (n=496) from 3 centres who entered a substudy. 2.6% were found to have an EF<0.40. Additionally, an audit of charts identified that in 5285 patients a prerandomization and evaluation of ventricular function had been conducted. Only 409 (7.7%) were documented to have low EF and none had heart failure prior to randomization. A separate analysis of those documented to have a preserved EF (n=4876) is provided. After obtaining written informed consent, all eligible patients entered a run in phase where they received 2.5mg Ramipril OD for 7-10 days followed by matching placebo for 10-14 days. Patients who were non compliant (<80% of pills taken), who experienced side effects, developed abnormal creatinine or potassium levels or those who withdrew consent were excluded. 9,541 were included ; 9,297 were randomized to receive Ramipril at 10mg/day or matching placebo, with 244 randomized to low dose (2.5mg/day) of Ramipril.

At randomization, patients were allocated to receive Ramipril at 2.5mg OD for one week, then 5mg OD for another 3 weeks, followed by 10mg once daily, or matching placebo. Additionally, all patients were randomized to vitamin E 400 IU/day or matching placebo. Follow-up visits occurred at 1 month, 6 months and then at 6 month intervals. At each visit, data were collected on events, compliance, and side effects leading to alteration of study medications. All primary and secondary events were documented on additional forms and were centrally adjudicated using standardized definitions.

Study organization: Patients were recruited over an 18 month period (Dec 1993 to June 1995) from centres in Canada (129), the USA (27), 14 Western European countries (76), Argentina and Brazil (30), and Mexico (5). Each institution's review board approved the protocol. The study was organized and coordinated by the Canadian Cardiovascular Collaboration Project Office located at the Preventive Cardiology and Therapeutics Research Program, Hamilton Health Sciences Corporation Research Centre, McMaster University, Hamilton, Canada. Adjunct project offices were located in London, England; São Paulo, Brazil and Rosario, Argentina. The responsibility for the overall study was undertaken by the Steering Committee.

Statistical Analyses: The study was originally designed to follow participants for a mean of 3.5 years. However, before the end of this period, the Steering Committee (blinded to any results) postulated a possible lag before treatment would have its full effects and recommended extension of follow up to 5 years. Assuming an event rate of 4% per year for 5 years, with 9,000 patients there would be 90% power to detect a 13.5% relative risk reduction utilizing a 2 sided alpha of 0.05, analyzed on an intention to treat basis. Survival curves were estimated using the Kaplan-Meier procedure and compared treatments utilizing a log-rank test. Because of the factorial design, all analyses were stratified for the randomization to vitamin E or control. Subground analyses were conducted utilizing tests for interaction in the Cox regression model. This model was also used for treatment effect estimates adjusted for any imbalances in key prognostic factors. The adjusted and unadjusted analyses provided virtually identical results, so only the unadjusted estimates are provided.

Interim analysis, data monitoring and early termination: An independent Data and Safety Monitoring Board (DSMB) monitored the progress of all aspects of the study. Four formal interim analyses were planned. The statistical monitoring boundary for benefit required crossing four standard deviations for the first half of the trial and three standard deviations in the second half. For harm the respective boundaries were three and two standard deviations respectively. The decision to stop or continue the trial would depend on a number of additional factors including consistency of results across key subgroups. On March 22^{nd} 1999, the independent DSMB recommended termination of the trial because of clear and persistent evidence of benefit of Ramipril which had consistently and clearly crossed the monitoring boundaries on two consecutive looks. (20% relative risk reduction in the primary outcome with 95% Cl of 12% to 28%, Z of -4.5; p=0.00001). The results of the trial were disclosed to the investigators at two meetings held on April 17^{th} and April 24^{th}. A cut off for all events for the main analysis was set for April 15^{th} 1999. Close our visits commenced on April 19^{th} and were scheduled to be completed by June 30^{th}, 1999.

### Example 2

### Patient characteristics:

Table 1 provides the baseline characteristics of patients entering the trial. Of note there were 2480 women (26.7%), 5128 individuals≥65 years (55.2%), 8160 with vascular disease (87.8%), 4355 with a history of hypertension (46.8%) and 3578 with diabetes (38.5%). This makes HOPE the largest trial of ACE-I in women, the elderly and among diabetics and with a sizeable number of high risk hypertensives.

**Table 1: Baseline Characteristics of the HOPE Study Patients**

| | Ramipril | Placebo |
|---|---|---|
| | n (%) | N (%) |
| No. Randomized | N = 4645 | N = 4652 |
| Mean Age | 66 (7) | 66 (7) |
| No. Women | 27.5 | 25.8 |
| History of Coronary artery disease | 3691 (79.5) | 3784 (81.3) |
| Myocardial infarction | 2410 (51.9) | 2482 (53.4) |
| ≤ 1 year | 452 (9.7) | 445 (9.6) |
| > 1 year | 1985 (42.7) | 2070 (44.5) |
| Stable angina pectoris | 2538 (54.6) | 2609 (56.1) |
| Unstable angina pectoris | 1179 (25.4) | 1188 (25.5) |
| CABG surgery | 1192 (25.7) | 1207 (25.9) |
| PTCA | 853 (18.4) | 806 (17.3) |
| Stroke or Transient ischemic attacks | 500 (10.8) | 513 (11.0) |
| Peripheral Vascular disease | 1963 (42.3) | 2083 (44.8) |
| Hypertension | 2212 (47.6) | 2143 (46.1) |
| Diabetes | 1808 (38.9) | 1770 (38.0) |
| Known elovated total cholesterol | 3036 (65.4) | 3089 (66.4) |
| Known low LDL | 842 (18.1) | 882 (19.0) |
| Current cigarette smoking | 645 (13.9) | 674 (14.5) |
| Drugs at Baseline: | | |
| Beta Blockers | 1820 (39.2) | 1853 (39.8) |
| Aspirin/ other antiplatelets | 3497 (75.3) | 3577 (76.9) |
| Lipid lowering agent | 1318 (28.4) | 1340 (28.8) |
| Diuretics | 713 (15.3) | 706 (15.2) |
| Calcium channel blockers | 2152 (46.3) | 2228 (47.9) |
| ECG left ventricular hypertrophy | 378 (8.1) | 405 (8.7) |
| No. With microalbuminuria | 955 (20.6) | 1008 (21.7) |
| Blood Pressure | 139/79 | 139/79 |
| Heart Rate | 69 | 69 |
| Body Mass Index | 28 | 28 |

CABG = Coronary artery bypass graft surgery, PTCA = Percutaneous transhuminal coronary angioplasty, No. = number, LDL = Low density Lipoprotein. Peripheral vascular disease includes claudication, history of peripheral arterial disease or ankle-arm BP ratio of <0.90.

### Example 3

### Compliance

Among those allocated to the Ramipril group, the proportion taking study or open label ACE-I was 87.4% at 1 year, 85.2% at 2 years, 82.2% at 3 years, 75.5% at 4 years and 78.3% at the final visit. 82.9% were receiving 10mg of Ramipril at 1 year, 74.8% at 2 years, 71.0% at 3 years, 62.8% at 4 years and 64.6% at last visit. Among those allocated to placebo, the proportion on open label ACE-I was 3.4%, 6.0%, 8.1%, 10.7% and 12.7% respectively. At the end of the study 1.6% of Ramipril patients and 1.9% of placebo patients were receiving an angiotensin-2 receptor antagonist. The most common reasons for discontinuing blinded medication are outlined in Table 2. More patients in the Ramipril group stopped medications for cough (7.2% v 1.7%) or hypertension (1.8% v 1.4%). By contrast, more placebo patients stopped blinded medication for uncontrolled hypertension (0.3% v 0.6%) or for a clinical event (1.9% v 2.4%). The proportions of patients receiving non-study ACE-I for heart failure was 3.3% in the active group and 4.5% in the placebo group, for proteinuria was 1.4% v 1.6%, and for control of hypertension 4.4% v 6.2%. The use of open label A-2 receptor antagonists in both groups was low (1.6% v 1.9%) but the reasons reflect a pattern similar to the use of ACE-inhibitors (heart failure 0.6% v 0.8%, hypertension 1.1 % v 1.3%).

**Table 2: Reasons for discontinuing blinded medication**

| | Ramipril | Placebo |
|---|---|---|
| No. Randomized | 4645 | 4652 |
| No. Stopping at any time* | 1370 (33.0) | 1247 (30.7) |
| No. Permanently discontinuing* | 1207 (29.1) | 1087 (26.7) |
| Reasons for stopping | | |
| Cough | 335 (7.2 %) | 81 (1.7 %) |
| Hypotension/dizziness | 82 (1.8 %) | 65 (1.4 %) |
| Angioedema | 16 (0.3 %) | 12 (0.3 %) |
| Uncontrolled Hypertension | 16 (0.3 %) | 30 (0.6 %) |
| Clinical Events | 90 (1.9 %) | 113 (2.4 %) |
| Non-study ACE-I | 124 (2.7 %) | 187 (4.0 %) |
| Reasons for using open label ACE-I: | | |
| Heart Failure | 231 (5.0 %) | 320 (6.9 %) |
| Proteinuria | 63 (1.4) | 73 (1.6 %) |
| Hypertension | 205 (4.4 %) | 289 (6.2 %) |

| | | |
|---|---|---|
| * % of alive | | |

### Example 4

### Blood Pressure

The BP at entry was 139/79 in both groups. This decreased to 133/76 in the active group and 137/78 in the control group at 1 month, 135/76 and 138/78 at 2 years and 136/76 and 139/77 at the end of the study.

### Example 5

### Primary Outcomes and total mortality (Table 3)

There were 646 patients in the Ramipril group (13.9%) who suffered CV death, MI or stroke compared to 816 (17.5%) in the placebo group (RR of 0.78, 95% CI of 0.70-0.86; p=0.000002). In addition there were highly significant reductions separately in CV mortality (278 v 371, RR of 0.75, 95% CI of 0.64-0.87; p=0.0002), MI (453 v 559, RR of 0.80, 95% CI of 0.71-0.91; p=0.0005) and stroke (155 v 225, RR of 0.68, 95% CL of 0.56-0.84; p=0.0001). All cause mortality was also significantly reduced (476 v 567, RR of 0.83, 95% CI of 0.74-0.94; p=0.0035).

**Table 3: Primary Outcome and its Components in the HOPE Study**

| | Ramipril | Placebo | RRR (95 % CI) | Z | Log rank p |
|---|---|---|---|---|---|
| No. Randomized | 4645 | 4652 | | | |
| CV death, MI, Stroke* | 646 (13.9 %) | 816 (17.5 %) | 0.78 (0.70-0.86) | -4.75 | 0.000002 |
| CV death | 278 (6.0 %) | 371 (8.0 %) | 0.75 (0.64-0.87) | -3.72 | 0.0002 |
| MI | 453 (9.8 %) | 559 (12.0 %) | 0.80 (0.71-0.91) | -3.49 | 0.0005 |
| Strokes | 155 (3.3 %) | 225 (4.8 %) | 0.68 (0.56-0.84) | -3.70 | 0.0002 |
| Total mortality | 476 (10.3 %) | 567 (12.2 %) | 0.83 (0.74-0.94) | -2.92 | 0.0035 |

| | | | | | |
|---|---|---|---|---|---|
| *There were an additional 34/244 events with low dose Ramipril. Inclusion of these events leads to 13.9 % primary events with Ramipril v 17.5 % with placebo (RRR of 0.78, 95 % Cl of 0.70-0.86). Note that patients could have experienced more than one event. | | | | | |

### Example 6

### Secondary and other outcomes (Table 4)

There was a significant reduction in the number of patients undergoing revascularization procedures (742 v 854, RR of 0.85, 95% CI of 0.77-0.94; p=0.0013), and a trend to fewer HF hospitalizations (150 v 176; RR of 0.84, 95% CI of 0.68 to 1.05; p=0.13). However, there was no impact on hospitalizations for unstable angina. There were also significant reductions in the number of patients with cardiac arrests (37 v 58, RR=0.63, p.0.03) worsening angina (1104 v 1220, RR=0.88, p=0.003), new heart failure (343 v 435, RR=0.78, p=0.0005), new diagnosis of diabetes (108 v 157, RR=0.69, p=0.003), or those experiencing diabetic complications (319 v 378, RR=0.85, p=0.018).

**Table 4: Secondary and other Outcomes in the HOPE Study**

| | Ramipril | Placebo | RRR (95 % CI) | Z | Log rank p |
|---|---|---|---|---|---|
| No. Randomized | 4645 | 4652 | | | |

| Secondary Outcomes | | | | | |
|---|---|---|---|---|---|
| Revascularization* | 742 (16.0 %) | 854 (18.4 %) | 0.85 (0.77-0.94) | -3.22 | 0.0013 |
| Unstable angina hospitalization* | 564 (12.1 %) | 573 (12.3 %) | 0.98 (0.87-1.10) | - | n.s. |
| Diabetic Complications ⁺* | 298 (6.4 %) | 357 (7.7 %) | 0.83 (0.71-0.97) | -2.39 | 0.017 |
| Heart Failure Hospitalizations* | 150 (3.2 %) | 176 (3.8 %) | 0.84 (0.68-1.05) | -1.53 | 0.13 |

| Other Outcomes | | | | | |
|---|---|---|---|---|---|
| All Heart Failure** | 343 (7.4 %) | 435 (9.4 %) | 0.78 (0.67-0.90) | -3.51 | 0.0005 |
| Cardiac Arrests | 37 (0.8 %) | 58 (1.2 %) | 0.63 (0.42-0.96) | -2.19 | 0.03 |
| Worsening angina** | 1104 (23.8 %) | 1220 (26.2 %) | 0.88 (0.81-0.96) | -2.98 | 0.0029 |
| New diagnosis of diabetes | 108 (3,8 %) | 157 (5.5 %) | 0.69 (0.54-0.88) | -3.01 | 0.0026 |
| UA with ECG changes | 179 (3.4 %) | 185 (4.0 %) | 0.96 (0.78-1.18) | - | n.s. |

Centrally adjudicated events, ** Includes cases irrespective of hospitalization. ⁺ Diabetic complications include diabetic nephropathy, renal dialysis and laser therapy for diabetic retinopathy.

### Example 7

### Subgroup analysis (Table 5)

The beneficial impact on the primary outcome was consistently observed among diabetic and nondiabetic patients; females and males, those with and without evidence of vascular disease, those under and over 65 years, those with and without hypertension at baseline and those with and without microalbuminuria at baseline. In addition, there was a clear benefit among both groups of patients entering the study with or without evidence of coronary artery disease, with and without an MI and among those (n=4676) with a documented EF ≥0.40 (317/2339 v 427/2337, RR of 0.73, 95% CI of 0.63-0.84, p=0.00002).
Table 5: Impact of Ramipril compared to Placebo in Various Subgroups: Note the consistency of results and that the upper 95 % CI is less than 1 in most instances.

| | No. of Patients | Placebo Rate | Primary Composite Outcome RRR (95 % CI) |
|---|---|---|---|
| A) Prespecified Subgroups | | | |
| CVD + | 8160 | 18.5 | 078 (0.71 - 0.87) |
| CVD - | 1137 | 10.1 | 0.81 (0.56 - 1.20) |
| Diabetes+ | 3578 | 19.6 | 0.76 (0.65 - 0.89) |
| Diabetes- | 5719 | 16.3 | 0.79 (0.69 - 0.91) |

| B) Other Subgroups | | | |
|---|---|---|---|
| Age <65 | 4169 | 14.0 | 0.82 (0.70 - 0.98) |
| Age 65+ | 5128 | 20.5 | 0.75 (0.66 - 0.85) |
| Male | 6817 | 18.5 | 0.79 (0.70 - 0.89) |
| Female | 2480 | 14.7 | 0.76 (0.61 - 0.95) |
| Hypertension + | 4355 | 19.0 | 0.75 (0.65 - 0.87) |
| Hypertension - | 4942 | 16.3 | 0.81 (0.70 - 0.93) |
| CAD+ | 7475 | 18.4 | 0.80 (0.71 - 0.89) |
| CAD- | 1822 | 13.9 | 0.71 (0.54 - 0.93) |
| Prior MI+ | 4892 | 20.7 | 0.79 (0.69 - 0.90) |
| Prio MI- | 4405 | 14.0 | 0.77 (0.65 - 0.91) |
| Cerebro VD+ | 1013 | 25.5 | 0.73 (0.56 - 0.95) |
| Cerebro VD- | 8284 | 16.6 | 0.79 (0.71 - 0.88) |
| PVD+ | 4046 | 21.8 | 0.74 (0.64 - 0.86) |
| PVD- | 5251 | 14.1 | 0.84 (0.72 - 0.98) |
| MA+ | 1963 | 26.1 | 0.71 (0.59 - 0.86) |
| MA- | 7334 | 15.2 | 0.82 (0.72 - 0.92) |

| | | | |
|---|---|---|---|
| CVD = Cardiovascular disease, CAD = Coronary artery disease, | | | |
| MA = Microalbuminuria, PVD = Peripheral vascular disease | | | |

### Example 8

### Time Course of Benefit

The reduction in the primary outcome was evident within 1 year after randomization (167 v 197, RR of 0.85; 95% CI of 0.69 to 1.04), and became statistically significant at 2 years (323 v 396; RR of 0.81; 95% CI of 0.70 to 0.94. Conditioned on survival up to the prior year, the relative risk in the second year was 0.78, in the third 0.74, and 0.73 in the fourth year.

### DISCUSSION

The HOPE trial conclusively proves that Ramipril, an ACE-I, is beneficial in a broad range of patients without evidence of LV systolic dysfunction or HF who are at high risk of future cardiovascular events. There are clear reductions in each of mortality, MI and strokes. Coronary revascularizations, cardiac arrests and development of heart failure are also clearly reduced. Ramipril also reduces the risk of diabetic complications; and the development of diabetes among non-diabetics.

### Benefits of ACE-I Now Extend More Widely

These data substantially expand the population who would benefit from ACE-I and are complementary to previous studies in patients with low EF or HF and acute MI. The underlying rationale for the HOPE study was that ACE inhibition would prevent the events that related to ischemia and atherosclerosis, in addition to the heart failure and left ventricular dysfunction. To avoid potential confusion in the interpretation of the results of this study, we deliberately confined our trial to those without HF and excluded those with a known low EF. The study did include, however, the large number of individuals at risk of outcomes related to the progression of atherosclerosis and thrombotic vascular occlusion. Thus a broad range of patients with any manifestation of coronary artery disease (eg unstable angina, stable angina or previous revascularization), previous history of cerebrovascular disease or peripheral arterial disease were included. As a result, we have been able to demonstrate the value of ACE-I in a broad spectrum of patients with a range of clinical manifestations of atherosclerosis that increased the risk of CV death, MI or stroke. This approach is neither primary or secondary prevention, but is rather a high risk prevention strategy, which includes individuals with a high likelihood of a future event, rather than including patients solely by the presence of a specific risk factor or occurrence of a specific cardiovascular event. The present results attest to the success of this approach in identifying a high-risk population with a significant rate of CV endpoints who are likely to benefit from a therapy which prevents the progression of atherosclerosis or its complications. These findings along with those from previous trials are of major clinical importance and indicate that documentation of low EF on HF should not be a criterion to using ACE-I long term in patients who are at high risk of CV events, due to other clinical criteria.

There were 3578 diabetic patients entering our study, of whom 1100 had no clinical manifestations of CVD, and their risk of CV outcomes was lower by about half. Despite this, the RRR we observed was consistent with the overall benefit in the trial and among such diabetics the composite outcome of CV death, MI, stroke, heart failure, revascularization and diabetic complications was significantly reduced.

### Benefits of ACE-I Similar to Other Preventive Strategies

The magnitude of benefit of ACE-I is at least as large as that observed with other proven secondary prevention measures such as betablockers (Yusuf S et al., Prog Cardiovasc Dis 1985; 27(5): 335-371), aspirin (BMJ 1994; 308(6921): 81-106) and lipid lowering (Law M. Lipids and cardiovascular disease. Chpt 13 In: Yusuf S, Cairns JA, Camm AJ, Fallen EL, Gersh BJ. (Eds), Evidence Based Cardiology. London: BMJ Books, 1998. Pg. 191-205) over a 4 year treatment period. Given the broad population in HOPE and previous trials of ACE-I, the very clear evidence of benefit in addition to other effective therapies and high tolerance, ACE-I have a major role in CV prevention and treatment. The relative risk reduction in the first year of the trial was about 11 %, increasing to 22% (conditional RRR) in the 2^{nd} year, 26% in the 3^{rd} year and 27% in the 4^{th} year. These data indicate a very rapid emergence of benefit with increasing divergence in the second year which is at least maintained and perhaps increased in future years. This suggests that the benefits of ACE-I are likely to be sustained and perhaps enhanced on longer term treatment.

### Benefits are Additional to Concomitant Proven Medications

The benefits of Ramipril were observed among patients receiving a number of effective treatments such as aspirin, betablockers and lipid lowering agents indicating that ACE inhibition offers an additional approach to prevent atherothrombotic complications. Only a small part of the reductions in CV mortality, MI and strokes could be attributed to BP reduction as the majority of patients entering the study were not hypertensive (by conventional definitions) and the difference in BP reduction was extremely modest (-3 systolic /-2 mmHg diastolic). A 2mm reduction in diastolic BP reduction might at best explain about half in the reduction in stroke and about one-quarter of the reduction in myocardial infarction (Collins R. et al., Lancet 1990; 335(8693): 827-838). However, recent trials such as the Hypertension Optimum Treatment (Hansson L. et al., Lancet 1998; 351(9118): 1755-1762) have suggested that for high risk patients, eg diabetics, it may be beneficial to lower BP even within the "normal" range. Moreover, a recent reanalysis of the Framingham Heart Study based on 20 year BP data (Clarke R. et al., Am J Epidemiology 1999; 150(4): (In press) suggests that the degree of benefit expected from a lower BP may have been underestimated. Despite these considerations, it is possible that additional direct mechanisms of ACE-I on the heart or the vasculaturs is of importance. This includes antagonizing the direct effects of angiotensin-II on vasconstriction, vascular smooth muscle proliferation (Lonn EM et al. Circulation 1994; 90(4): 2056-2069) and plaque rupture (Schieffer B. et al., Circulation (in press)); improvement in vascular endothelial function, reduction of LV hypertrophy and enhanced fibrinolysis (Lonn EM et al. Circulation 1994; 90(4): 2056-2069).

Also a reduction in the number of patients developing or being hospitalized for heart failure was observed in patients with no evidence of impairment of LV systolic function. These data complement the SOLVD Prevention Trial in patients with low EF, and the SAVE trials (low EF early post-MI) which demonstrated that ACE-I prevent the development of heart failure; and the trials in patients with documented low EF and HF which indicated a reduction in hospitalization for heart failure. HOPE and these studies indicate that ACE-I are likely to be of value among patients who are at high risk of developing heart failure irrespective of the degree of left ventricular systolic dysfunction. One issue that should be considered is the extent to which the results may have been affected by inclusion of individuals with undiagnosed low EF. This is likely to be very low because a) a large substudy in 3 centres involving 468 consecutive patients indicated that only 2.6% had an EF below .40, b) an extensive chart audit identified only <5% of patients with a low EF prior to randomization; and c) clear benefit (RR of 0.73, 95% CI of 0.63 to 0.84; p=0.00002) was seen in the subgroup of patients (n = 4676) with documented preserved ventricular function and also in those without previous MI. (RR of 0.79, 95% CI of 0.69 to 0.90; p=0.0004)

### Possible Mechanisms of Benefit in Diabetes

A marked reduction in the number of patients developing diabetic complications and the number being newly diagnosed as having diabetes was observed. These effects may be mediated through improved insulin sensitivity or a decrease in hepatic clearance of insulin. The results are also consistent with the results of the recent Captopril Prevention Project trial (Hansson L. et al., Lancet 1999; 353(9153): 611-616), which indicated a reduction in newly diagnosed diabetes in patients randomized to captopril compared to a diuretic or beta blocker, and other trials indicating that the progression of diabetic nephropathy among type II diabetics treated with an ACE-inhibitor is reduced (Ruggenenti P. et al. Lancet 1999; 354 (9176): 359-364).

### Safety and Tolerability

The ACE-inhibitor Ramipril, was generally well tolerated in the trial. Apart from an increase in the number of patients stopping Ramipril for cough (excess of 5%), no other side effect was significantly more frequent. There was a small nonsignificant increase in the number of patients stopping medication for dizziness/hypotension (0.3%). The majority of patients (approximately 80%) remained on an ACE-I over the 4.2 year duration of the trial.

### Conclusion

The HOPE study clearly demonstrates that Ramipril, a long acting ACE-inhibitor, reduces mortality, MI, strokes, revascularization rates, cardiac arrests, new heart failure and diabetic complications in a broad spectrum of high risk patients. Treating 1000 patients with ACE-inhibitors for 4 years prevents 160 patients from experiencing any one of the above events.

The contents of all patents, patent applications, published articles, books, reference manuals and abstracts cited herein are hereby incorporated by reference in their entirety to more fully describe the state of the art to which the invention pertains.

As various changes can be made in the above-described subject matter without departing from the scope and spirit of the invention, it is intended that all subject matter contained in the above description, shown in the accompanying drawings, or defined in the appended claims, be interpreted as descriptive and illustrative, and not in a limiting sense. Many modifications and variations of the present invention are possible in light of the above teachings. It is therefore to be understood that within the scope of the appended claims, the invention may be practiced otherwise than as specifically described.

## Claims

1. The use of an inhibitor of the renin-angiotensin system or a pharmaceutically acceptable salt thereof in the manufacture of a medicament for the for the prevention of diabetes in a high risk patient with no evidence of left ventricular dysfunction or heart failure.

2. The use according to claim 1, wherein the high risk patient is a patient at risk having an cardiovascular event due to a manifest coronary heart disease, a history of transient ischaemic attacks or stroke or a history of peripheral vascular disease.

3. The use according to claims 1-2, where the inhibitor of the renin-angiotensin system or a pharmaceutically acceptable salt thereof is used together with an other antihypertensive, a cholesterol lowering agent, a diuretic or aspirin.

4. The use according to claim 3, wherein the antihypertensive is a calcium channel blocker or a beta blocker.

5. The use according to claim 3, wherein the cholesterol-lowering agent is a statin.

6. The use according to claim 5, wherein the statin is lovastatin, pravastatin, simvastatin or fluvastatin.

7. The use according to any of the claims 1 to 6, wherein the inhibitor of the renin-angiotensin system is an angiotensin converting enzyme inhibitor, an angiotensin II antagonist , or a pharmaceutically acceptable salt of any of these.

8. The use according to claim 7, wherein the angiotensin converting enzyme inhibitor is omapatrilat, MDL100240, alacepril, benazepril, captopril, cilazapril, delapril, enalapril, enalaprilat, fosinopril, fosinoprilat, imidapril, lisinopril, perindopril, quinapril, ramipril, ramiprilat, temocapril, trandolapril, trandolaprilat, ceranapril, moexipril, quinaprilat and spirapril or a pharmaceutically acceptable salt thereof.

9. The use according to claim 8, wherein the angiotensin converting enzyme inhibitor is ramipril.

10. The use according to claim 7, wherein the angiotensin II antagonist is saralasin acetate, candesartan cilexetil, valsartan, candesartan, losartan potassium, eprosartan, irbesartan, tasosartan, or telmisartan or a pharmaceutically acceptable salt thereof.
